# EUROPÄISCHE PATENTANMELDUNG

(11) **EP 4 703 003 A1**
(43) Veröffentlichungstag der Anmeldung: **04.03.2026**
(21) Anmeldenummer: 24196765.2
(22) Anmeldetag: 27.08.2024
(51) Int. Cl.: A61N 1/36

(54) **SYSTEM ZUM STIMULIEREN WENIGSTENS EINES MUSKELS**

(71) Anmelder: Fraunhofer-Gesellschaft zur Förderung der angewandten Forschung e.V., 80686 München (DE); Rheinisch-Westfälische Technische Hochschule (RWTH) Aachen, 52062 Aachen (DE)
(72) Erfinder: Brandl, Christopher, Aachen (DE); Vrontos, Apostolos, Aachen (DE)
(74) Vertreter: Pfenning, Meinig & Partner mbB

(57) **Zusammenfassung**

Die Erfindung betrifft ein System (10) zum Stimulieren wenigstens eines Muskels mittels elektrischer Stimulation. Das System (10) umfasst eine Steuereinheit (2) die eingerichtet ist, während einer ersten Kalibrierungsphase Kalibrierungsdaten wenigstens eines Probanden (1) zu erfassen und zu speichern, während einer zweiten Kalibrierungsphase Kalibrierungsdaten eines Nutzeres (7) zu erfassen und zu speichern, und während einer Anwendung eine dem Nutzer (7) verabreichte elektrische Stimulation basierend auf den während der ersten und der zweiten Kalibrierungsphase erfassten und gespeicherten Kalibrierungsdaten des Probanden (1) und des Nutzers (7) einzustellen. Die Erfindung betrifft ferner ein Verfahren zum Ansteuern wenigstens eines Elektrodenpaares (3).

## Beschreibung

Die Erfindung betrifft ein System zum Stimulieren wenigstens eines Muskels und ein Verfahren zum Ansteuern eines Elektrodenpaares. Anwendungen der Erfindung liegen beispielsweise auf dem Gebiet haptischer Mensch-Maschine Schnittstellen, z. B. auf dem Gebiet der Erzeugung einer virtuellen Realität, des Sports, insbesondere des Trainings, oder der Arbeitssicherheit, der Fahrzeugführung oder der Systembedienung.

Es ist bekannt, dass ein Muskel eines menschlichen oder tierischen Körpers mittels auf der Haut angebrachter Elektroden elektrisch stimuliert werden kann, um den Muskel z. B. zur Kontraktion zu veranlassen. Bekannt sind beispielsweise Anwendungen im Bereich Muskeltraining und medizinischer Therapie. Gewöhnlich werden Stimulationsparameterwerte wie z. B. eine Amplitude, eine Frequenz oder eine Pulsbreite elektrischer Stimulationspulse dabei über eine manuelle Eingabeschnittstelle eingestellt. Dies kann z. B. eine Auswahl eines einer Vielzahl von vordefinierten Stimulationsprofilen beinhalten. Dieses Vorgehen eignet sich jedoch in der Regel nicht für Anwendungen, die eine adaptive und gezielte Erzeugung von Muskelkräften und propriozeptiven Empfindungen erfordern, weil u. a. quantitative Zielwerte für die Stellgrößen der elektrischen Stimulatioin nicht oder nur mit großem Aufwand manuell bestimmbar sind und eine Adaption der Stellgrößen der elektrischen Stimulation normalerweise nicht schnell genug und in Echtzeit durchgeführt werden kann.

In Galofaro, E.; D'Antonio, E.; Lotti, N.; Masia, L. Rendering immersive haptic force feedback via neuromuscular electrical stimulation. Sensors 2022, 22, 5069 (im Folgenden: Galofaro et al.) stellen die Autoren einen Versuchsaufbau vor, der neuromuskuläre elektrische Stimulation (NMES) einsetzt, um in einem Muskel durch elektrische Stimulation des Antagonisten dieses Muskels das haptische Gefühl zu erzeugen, belastet zu werden. Dazu wenden die Autoren ein subjektspezifisches biomechanisches Modell an, das das Drehmoment des Ellenbogens beim Heben von Gegenständen abschätzt, um dann durch eine geeignete elektrische Muskelstimulation ein entsprechendes haptisches Gefühl hervorzurufen.

Gemäß Galofaro et al. wurde dazu für jede Versuchsperson zunächst eine Kalibrierung des Stimulationssystems durchgeführt. Während der Kalibrierung wurden der Bizeps und der Trizeps der jeweiligen Versuchsperson mittels NMES stimuliert, wobei der NMES-Parameter Stimulationspulsbreite (Pulse Width oder PW) variiert wurde, und für jeden von zehn Werten des NMES-Parameters Stimulationspulsbreite wurde die von dem jeweiligen Muskel der Versuchsperson erzeugte Kraft mittels eines Kraftsensors gemessenen. In einem Versuchsszenario wurde die Versuchsperson dann in der Anwendung einer virtuellen Realität (VR) mittels NMES stimuliert, um bei ihr das haptische Gefühl zu erzeugen, das sie beim Heben eines realen Gewichts einer vorgegebenen Masse empfinden würde. Dabei wurde gemäß Galofaro et el. der NMES-Parameter Stimulationspulsbreite bei der elektrischen Stimulation einer Versuchsperson in der VR-Anwendung jeweils nur basierend auf den Kalibrierungsdaten derselben Versuchsperson bestimmt.

Um das Heben eines Gewichts einer vorgegebenen Masse in einer VR-Anwendung mittels NMES mit möglichst guter Genauigkeit simulieren zu können, muss bei dem von Galofaro et al. vorgeschlagenen System also für jede Versuchsperson bzw. für jeden Nutzer eine vergleichsweise zeit- und kostenintensive Kalibrierung durchgeführt werden. Dies macht das System vergleichsweise teuer und wenig anwenderfreundlich.

Der vorliegenden Erfindung liegt somit die Aufgabe zugrunde, ein System zur Muskelstimulation zu entwickeln, das für eine möglichst große Anzahl von Nutzern eine gewünschte muskuläre Antwort mit möglichst guter Genauigkeit erzielt und dabei möglichst anwenderfreundlich und kostengünstig ist. Ferner soll ein entsprechendes Verfahren zum Ansteuern eines Elektrodenpaares angegeben werden.

Diese Aufgabe wird durch ein System und durch ein Verfahren gemäß den unabhängigen Ansprüchen gelöst. Spezielle Ausführungsformen sind in den Unteransprüchen beschrieben.

Vorgeschlagen wird also ein System zum Stimulieren wenigstens eines Muskels mittels elektrischer Stimulation, umfassend:
wenigstens ein Elektrodenpaar zum Verabreichen elektrischer Stimulation und
eine mit dem wenigstens einen Elektrodenpaar verbindbare Steuereinheit zum Ansteuern des wenigstens einen Elektrodenpaares,
wobei die Steuereinheit eingerichtet ist,
ein Elektrodenpaar des wenigstens einen Elektrodenpaares während einer ersten Kalibrierungsphase zum Stimulieren jeweils wenigstens eines Muskels eines oder mehrerer Probanden derart anzusteuern, dass ein erster Stimulationsparameter jeweils wenigstens einen oder mehrere erste Stimulationsparameterwerte annimmt,
ein Elektrodenpaar des wenigstens einen Elektrodenpaares während einer zweiten Kalibrierungsphase zum Stimulieren wenigstens eines Muskels eines von dem oder den Probanden verschiedenen Nutzers derart anzusteuern, dass ein zweiter Stimulationsparameter wenigstens einen oder mehrere zweite Stimulationsparameterwerte annimmt, und
basierend auf dem wenigstens einen oder den mehreren ersten Stimulationsparameterwerten und basierend auf dem wenigstens einen oder den mehreren zweiten Stimulationsparameterwerten wenigstens einen oder mehrere dritte Stimulationsparameterwerte eines dritten Stimulationsparameters zu bestimmen oder zu schätzen und ein Elektrodenpaar des wenigstens einen Elektrodenpaares während einer Anwendung zum Stimulieren wenigstens eines Muskels des Nutzers derart anzusteuern, dass der wenigstens eine dritte Stimulationsparameter den wenigstens einen oder die mehreren dritten Stimulationsparameterwerte annimmt.

Ferner wird ein Verfahren zum Ansteuern eines Elektrodenpaares vorgeschlagen, insbesondere zum Stimulieren wenigstens eines Muskels mittels elektrischer Stimulation. Das Verfahren kann mit dem zuvor beschriebenen System durchgeführt werden. Das Verfahren umfasst wenigstens die folgenden Schritte:
während einer ersten Kalibrierungsphase Ansteuern wenigstens eines Elektrodenpaares zum Stimulieren jeweils wenigstens eines Muskels eines oder mehrerer Probanden derart, dass ein erster Stimulationsparameter jeweils wenigstens einen oder mehrere erste Stimulationsparameterwerte annimmt,
während einer zweiten Kalibrierungsphase Ansteuern wenigstens eines Elektrodenpaares zum Stimulieren wenigstens eines Muskels eines von dem oder den Probanden verschiedenen Nutzers derart, dass ein zweiter Stimulationsparameter wenigstens einen oder mehrere zweite Stimulationsparameterwerte annimmt,
Bestimmen oder Schätzen wenigstens eines oder mehrerer dritter Stimulationsparameterwerte eines dritten Stimulationsparameters basierend auf dem wenigstens einen oder den mehreren ersten Stimulationsparameterwerten und basierend auf dem wenigstens einen oder den mehreren zweiten Stimulatioparameterwerten und
während einer Anwendung Ansteuern wenigstens eines Elektrodenpaares zum Stimulieren wenigstens eines Muskels des Nutzers derart, dass der dritte Stimulationsparameter den wenigstens einen oder die mehreren dritten Stimulationsparameterwerte annimmt.

Dadurch, dass der wenigstens eine dritte Stimulationsparameterwert basierend auf dem wenigstens einen zweiten Stimulationsparameterwert, der mit dem Nutzer assoziiert ist, und zusätzlich basierend auf dem wenigstens einen ersten Stimulationsparameterwert, der mit dem von dem Nutzer verschiedenen Probanden assoziiert ist, bestimmt oder geschätzt wird, ist es möglich, ein beim Ansteuern des Elektrodenpaares während der Anwendung gewünschtes Ergebnis, z. B. die Generierung einer Kraft einer vorgegebenen Größe durch einen oder mehrere stimulierte Muskeln des Nutzers oder die Generierung einer Kraftempfindung einer vorgegebenen Größe durch den Nutzer, mit guter Genauigkeit zu erzielen. Ebenso ermöglicht das Bestimmen oder Schätzen des wenigstens einen dritten Stimulationsparameterwertes basierend auf dem mit dem Probanden assoziierten wenigstens einen ersten Stimulationsparameterwert typischerweise eine Vereinfachung und/oder Abkürzung der zweiten Kalibrierungsphase, da während der zweiten Kalibrierungsphase im Vergleich mit bekannten Systemen und Verfahren gewöhnlich eine geringere Anzahl an Werten des wenigstens einen zweiten Stimulationsparameters aufgenommen werden kann, ohne dass sich die Genauigkeit eines bei der Anwendung gewünschten Ergebnisses gegenüber bekannten Systemen und Verfahren dadurch verschlechtert.

Typischerweise charakterisiert der wenigstens eine erste Stimulationsparameter eine mittels des Elektrodenpaares während der ersten Kalibrierungsphase verabreichte elektrische Stimulation, z. B. in Gestalt elektrischer Stimulationspulse. Typischerweise charakterisiert der wenigstens eine zweite Stimulationsparameter eine mittels des Elektrodenpaares während der zweiten Kalibrierungsphase verabreichte elektrische Stimulation, z. B. in Gestalt elektrischer Stimulationspulse. Und typischerweise charakterisiert der wenigstens eine dritte Stimulationsparameter eine mittels des Elektrodenpaares während der Anwendung verabreichte elektrische Stimulation, z. B. in Gestalt elektrischer Stimulationspulse.

Der erste Stimulationsparameter, der zweite Stimulationsparameter und der dritte Stimulationsparameter können jeweils einen der folgenden Parameter umfassen: eine Amplitude elektrischer Stimulationspulse, z. B. eine Amplitude einer elektrischen Stromstärke oder eine Amplitude einer elektrischen Spannung elektrischer Stimulationspulse; eine Frequenz elektrischer Stimulationspulse; eine Pulsdauer elektrischer Stimulationspulse; eine Gesamtdauer der verabreichten elektrischen Stimulation; oder eine Gesamtenergie der verabreichten elektrischen Stimulation.

Der erste Stimulationsparameter kann gleich dem zweiten Stimulationsparameter und gleich dem dritten Stimulationsparameter sein. Der erste Stimulationsparameter, der zweite Stimulationsparameter und der dritte Stimulationsparameter können also z. B. jeweils denselben der im vorhergehenden Absatz aufgezählten Parameter umfassen oder enthalten, also z. B. jeweils eine Amplitude elektrischer Stimulationspulse etc. Ebenso ist es denkbar, dass der erste Stimulationsparameter, der zweite Stimulationsparameter und der dritte Stimulationsparameter oder wenigstens zwei von ihnen verschiedene Parameter umfassen oder enthalten. Beispielsweise ist es denkbar, dass der erste Stimulationsparameter eine Amplitude während der ersten Kalibrationsphase verabreichter elektrischer Stimulationspulse enthält und dass der zweite Stimulationsparameter eine Stimulationspulsbreite während der zweiten Kalibrationsphase verabreichter elektrischer Stimulationspulse enthält.

Wenn der erste Stimulationsparameter gleich dem zweiten Stimulationsparameter ist, können der wenigstens eine erste Stimulationsparameterwert und der wenigstens eine zweite Stimulationsparameterwert derart gewählt werden, dass wenigstens einer des wenigstens einen ersten Stimulationsparameterwertes von wenigstens einem des wenigstens einen zweiten Stimulationsparameterwertes verschieden ist. So kann z. B. eine möglichst große Variabilität der Stimulationsparameterwerte, die zur Bestimmung oder Schätzung des wenigstens einen dritten Stimulationsparameterwertes herangezogen werden, gewährleistet werden.

Der wenigstens eine erste Stimulationsparameterwert kann einen kleinsten Wert des ersten Stimulationsparameters umfassen, der den wenigstens einen Muskel des Probanden während der ersten Kalibrierungsphase zu einer Kontraktion veranlasst. Ebenso kann der wenigstens eine zweite Stimulationsparameterwert einen kleinsten Wert des zweiten Stimulationsparameters umfassen, der den wenigstens einen Muskel des Nutzers während der zweiten Kalibrierungsphase zu einer Kontraktion veranlasst. Alternativ oder zusätzlich kann der wenigstens eine erste Stimulationsparameterwert einen größten Wert des ersten Stimulationsparameters umfassen, der den wenigstens einen Muskel des Probanden während der ersten Kalibrierungsphase noch ohne geringfügiges Unwohlsein des Probanden zu einer Kontraktion veranlasst. Und alternativ oder zusätzlich kann der wenigstens eine zweite Stimulationsparameterwert einen größten Wert des zweiten Stimulationsparameters umfassen, der den wenigstens einen Muskel des Nutzers während der zweiten Kalibrierungsphase noch ohne geringfügiges Unwohlsein des Probanden zu einer Kontraktion veranlasst. Es hat sich herausgestellt, dass diese Werte gut geeignet sind, um die Reaktion eines oder mehrerer Muskeln auf elektrische Stimulation zu charakterisieren und um den wenigstens einen dritten Stimulationsparameterwert zu bestimmen oder zu schätzen.

Das System kann ferner eine mit der Steuereinheit verbundene oder verbindbare Eingabeeinheit zum Eingeben wenigstens eines oder mehrerer erster physischer Parameterwerte wenigstens eines oder mehrerer physischer Parameter des oder der Probanden während der ersten Kalibrierungsphase und zum Eingeben wenigstens eines oder mehrerer zweiter physischer Parameterwerte wenigstens eines oder mehrerer physischer Parameter des Nutzers umfassen. Der physische Parameter des Probanden kann auch individuelle Bestimmungsgröße menschlicher Leistung des Probanden genannt werden, und der physische Parameter des Nutzers kann auch individuelle Bestimmungsgröße menschlicher Leistung des Nutzers genannt werden. Der wenigstens eine oder die mehreren physischen Parameter des Probanden und der wenigstens eine oder die mehreren physischen Parameter des Nutzers können denselben oder dieselben physischen Parameter umfassen.

Die Steuereinheit kann dann eingerichtet sein, den wenigstens einen dritten Stimulationsparameterwert zusätzlich basierend auf dem wenigstens einen ersten physischen Parameterwert und basierend auf dem wenigstens einen zweiten physischen Parameterwert zu bestimmen oder zu schätzen. Mit anderen Worten kann der wenigstens eine dritte Stimulationsparameterwert zusätzlich basierend auf dem wenigstens einen ersten physischen Parameterwert und basierend auf dem wenigstens einen zweiten physischen Parameterwert bestimmt oder geschätzt werden. Die zusätzliche Berücksichtigung des wenigstens einen ersten physischen Parameterwertes und des wenigstens einen zweiten physischen Parameterwertes bei der Bestimmung oder Schätzung des wenigstens einen dritten Stimulationsparameterwertes kann es ermöglichen, das mit der Wahl des wenigstens einen dritten Stimulationsparameterwertes bei der Anwendung gewünschte Ergebnis mit besonders guter Genauigkeit zu erzielen. Ebenso kann dadurch gewöhnlich die Anzahl der während der zweiten Kalibrierungsphase aufgenommenen Werte verringert werden, was die Durchführung der zweiten Kalibrierungsphase oft erheblich vereinfachen und verkürzen kann.

Der wenigstens eine physische Parameter des Probanden und/oder der wenigstens eine physische Parameter des Nutzers können jeweils einen, mehrere oder alle der folgenden Parameter umfassen:
- eine Bezeichnung eines während der ersten Kalibrierungsphase und/oder während der zweiten Kalibrierungsphase stimulierten und/oder zu stimulierenden Muskels des Probanden und/oder des Nutzers,
- die Körpergröße des Probanden und/oder des Nutzers,
- das Körpergewicht des Probanden und/oder des Nutzers,
- das Lebensalter des Probanden und/oder des Nutzers,
- das Geschlecht des Probanden und/oder des Nutzers,
- den Körperfettantei des Probanden und/oder des Nutzers,
- eine Knochenmasse des Probanden und/oder des Nutzers,
- einen Körperflüssigkeitsanteil des Probanden und/oder des Nutzers,
- eine Muskelmasse des Probanden und/oder des Nutzers,
- wenigstens ein Größenmaß wenigstens eines Körperteils des Probanden und/oder des Nutzers, z. B. einen Oberarmumfang, einen Halsumfang, einen Bauchumfang oder dergleichen,
- eine maximal generierbare Kraft durch willentliche (im Fachjargon auch: willkürliche) Aktivierung eines Muskels des Probanden und/oder des Nutzers,
- ein oder mehrere Werte einer infolge der Stimulation mit dem wenigstens einen ersten Stimulationsparameterwert während der ersten Kalibrierungsphase durch den wenigstens einen Muskel des Probanden erzeugten Kraft,
- ein oder mehrere Werte einer infolge der Stimulation mit dem wenigstens ersten Stimulationsparameterwert während der ersten Kalibrierungsphase von dem Probanden empfundenen Kraft,
- ein oder mehrere Werte einer infolge der Stimulation mit dem wenigstens einen zweiten Stimulationsparameterwert während der zweiten Kalibrierungsphase durch den wenigstens einen Muskel des Nutzers erzeugten Kraft,
- ein oder mehrere Werte einer infolge der Stimulation mit dem wenigstens einen zweiten Stimulationsparameterwert während der zweiten Kalibrierungsphase von dem Nutzer empfundenen Kraft.

Die Steuereinheit kann eingerichtet sein, den wenigstens einen dritten Stimulationsparameterwert mittels maschinellen Lernens zu bestimmen oder zu schätzen.

Das hier vorgeschlagene Verfahren kann also ferner die folgenden Schritte enthalten:
Bestimmen oder Schätzen des wenigstens einen dritten
Stimulationsparameterwertes mittels maschinellen Lernens.

Beispielsweise kann die Steuereinheit eingerichtet sein, basierend auf dem wenigstens einen ersten Stimulationsparameterwert und basierend auf dem wenigstens einen zweiten Stimulationsparameterwert eine Tabelle und/oder eine mathematische Vorschrift, z. B. in Form einer mathematischen Funktion oder Abbildung, zu erzeugen oder zu schätzen, die jedem einer Vielzahl von Stimulationsparameterwerten des dritten Stimulationsparameters jeweils einen Wert einer Kraft oder einer Muskelkontraktion (meßbar z. B. mittels Elektromyographie, physikalische Einheit: Volt) zuordnet, die während der Anwendung beim Stimulieren des wenigstens eines Muskels des Nutzers mit dem jeweiligen Stimulationsparameterwert des dritten Stimulationsparameters durch diesen wenigstens einen Muskel des Nutzers generierbar ist und/oder durch den Nutzer empfindbar ist. Die Steuereinheit kann dann eingerichtet sein, den wenigstens einen dritten Stimulationsparameterwert zusätzlich basierend auf Einträgen der Tabelle oder basierend auf der mathematischen Vorschrift zu bestimmen oder zu schätzen.

Das hier vorgeschlagene Verfahren kann also ferner die folgenden Schritte enthalten:
Erzeugen oder Schätzen einer Tabelle und/oder einer mathematischen Vorschrift, z. B. in Form einer mathematischen Funktion oder Abbildung, basierend auf dem wenigstens einen ersten Stimulationsparameterwert und basierend auf dem wenigstens einen zweiten Stimulationsparameterwert, wobei die Tabelle und/oder die mathematische Funktion jedem einer Vielzahl von Stimulationsparameterwerten des dritten Stimulationsparameters jeweils einen Wert einer Kraft oder einer Muskelkontraktion (meßbar z. B. mittels Elektromyographie, physikalische Einheit: Volt) zuordnet, die während der Anwendung beim Stimulieren des wenigstens eines Muskels des Nutzers mit dem jeweiligen Stimulationsparameterwert des dritten Stimulationsparameters durch diesen wenigstens einen Muskel des Nutzers generierbar ist und/oder durch den Nutzer empfindbar ist, und
Bestimmen oder Schätzen des wenigstens einen dritten Stimulationsparameterwertes zusätzlich basierend auf Einträgen der Tabelle und/oder basierend auf der mathematischen Vorschrift.

Die Steuereinheit kann ferner eingerichtet sein, die Tabelle und/oder die mathematische Vorschrift ferner basierend auf dem wenigstens einen ersten physischen Parameterwert des Probanden und basierend auf dem wenigstens einen zweiten physischen Parameterwert des Nutzers zu erzeugen oder zu schätzen.

Das hier vorgeschlagene Verfahren kann also ferner die folgenden Schritte enthalten:
Erzeugen oder Schätzen der Tabelle und/oder der mathematischen Vorschrift ferner basierend auf dem wenigstens einen ersten physischen Parameterwert des Probanden und basierend auf dem wenigstens einen zweiten physischen Parameterwert des Nutzers.

Z. B. kann die Steuereinheit eingerichtet sein, die in der Tabelle enthaltenen Werte und/oder die mathematische Vorschrift durch Interpolation und/oder durch Extrapolation und/oder durch wenigstens einen Fit zu bestimmen, zu schätzen oder zu erzeugen.

Das hier vorgeschlagene Verfahren kann also ferner die folgenden Schritte enthalten:
Bestimmen oder Schätzen der in der Tabelle enthaltenen Werte und/oder Erzeugen der mathematischen Vorschrift durch Interpolation und/oder durch Extrapolation und/oder durch wenigstens einen Fit.

Die Steuereinheit kann eingerichtet sein, zur Bestimmung oder Schätzung des wenigstens einen dritten Stimulationsparameters nur erste Stimulationsparameterwerte und ggf. zusätzlich erste physische Parameterwerte solcher Probanden heranzuziehen, die dem Nutzer in Bezug auf die während der ersten Kalibrierungsphase und während der zweiten Kalibrierungsphase verwendeten oder erfassten ersten Stimulationsparameterwerte und ggf. zusätzlich in Bezug auf die während der ersten Kalibrierungsphase und während der zweiten Kalibrierungsphase verwendeten oder erfassten ersten und/oder zweiten physischen Parameterwerte besonders ähnlich sind. Dies kann die Genauigkeit eines gewünschten Ergebnisses, dass mit der Stimulation des Nutzers während der Anwendung mit dem wenigstens einen dritten Stimulationsparameterwert erzielt werden soll, weiter verbessern.

Zu diesem Zweck kann die Steuereinheit z. B. eingerichtet sein, den wenigstens einen dritten Stimulationsparameterwert mittels eines k-nächste-Nachbarn-Algorithmus zu bestimmen oder zu schätzen, wobei k eine natürliche Zahl größer oder gleich Eins ist. Insbesondere kann k z. B. den Wert jeder beliebigen natürlichen Zahl zwischen 1 und 10, zwischen 1 und 20 oder zwischen 1 und 50 annehmen.

Das hier vorgeschlagene Verfahren kann also ferner die folgenden Schritte enthalten:
Bestimmen oder Schätzen des wenigstens einen dritten Stimulationsparameterwertes mittels eines k-nächste-Nachbarn-Algorithmus, wobei k eine natürliche Zahl größer oder gleich Eins ist und z. B. den Wert jeder beliebigen natürlichen Zahl zwischen 1 und 10, zwischen 1 und 20 oder zwischen 1 und 50 annehmen kann.

Beispielsweise kann die Steuereinheit eingerichtet sein, dann, wenn die erste Kalibrationsphase für eine Vielzahl von Probanden durchgeführt wurde und der wenigstens eine erste Stimulationsparameterwert und ggf. der wenigstens eine erste physische Parameterwert jeweils für eine Vielzahl von Probanden vorliegen, zur Bestimmung oder Schätzung des wenigstens einen dritten Stimulationsparameterwertes nur diejenigen ersten Stimulationsparameterwerte und ggf. diejenigen ersten physischen Parameterwerte heranzuziehen, die jeweils einem Probanden aus einer Teilmenge aller Probanden zugeordnet sind, wobei die Teilmenge die Mächtigkeit k hat und nur diejenigen Probanden enthält, deren Abstand vom Nutzer in einem durch den ersten Stimulationsparameter und ggf. den wenigstens einen ersten physischen Parameter aufgespannten Parameterraum jeweils kleiner ist, als die Abstände vom Nutzer derjenigen Probanden in demselben Parameterraum, die kein Element der Teilmenge sind.

Das hier vorgeschlagene Verfahren kann also ferner die folgenden Schritte enthalten:
wenn die erste Kalibrationsphase für eine Vielzahl von Probanden durchgeführt wurde und der wenigstens eine erste Stimulationsparameterwert und ggf. der wenigstens eine erste physische Parameterwert jeweils für eine Vielzahl von Probanden vorliegen, Bestimmen oder Schätzen des wenigstens einen dritten Stimulationsparameterwertes basierend nur auf denjenigen ersten Stimulationsparameterwerten und ggf. zusätzlich basierend nur auf denjenigen ersten physischen Parameterwerten, die jeweils einem Probanden aus einer Teilmenge aller Probanden zugeordnet sind, wobei die Teilmenge die Mächtigkeit k hat und nur diejenigen Probanden enthält, deren Abstand vom Nutzer in einem durch den ersten Stimulationsparameter und ggf. den wenigstens einen ersten physischen Parameter aufgespannten Parameterraum jeweils kleiner ist, als die Abstände vom Nutzer derjenigen Probanden in demselben Parameterraum, die kein Element der Teilmenge sind.

Das System kann ferner ein mit der Steuereinheit verbundenes oder verbindbares Bewegungs-Erfassungssystem zum Erfassen einer Haltung und/oder einer Bewegung wenigstens eines Teils eines Körpers des Nutzers umfassen. Die Steuereinheit kann dann eingerichtet sein, den wenigstens einen dritten Stimulationsparameterwert zusätzlich basierend auf einer durch das Bewegungs-Erfassungssystem erfassten Haltung und/oder Bewegung wenigstens eines Teils des Körpers des Nutzers zu bestimmen oder zu schätzen. Das Bewegungs-Erfassungssystem kann z. B. eine oder mehrere mit der Steuereinheit verbundene oder verbindbare Kameras und/oder eine Vielzahl von mit der Steuereinheit verbundenen oder verbindbaren Sensoren umfassen. Die Sensoren können z. B. inertiale Messeinheiten (inertial measurement units oder IMUs) umfassen, die an Körperteilen des wenigstens einen Probanden und des Nutzers, z. B. an Armen, Händen, Beinen, Füßen, Rumpf, Kopf, anbringbar sind. Die Steuereinheit kann dann z. B. eingerichtet sein, basierend auf mittels des Bewegungs-Erfassungssystems erfassten Haltungs- und/oder Bewegungssignalen jeweils Positionen, Ausrichtungen und Geschwindigkeiten verschiedener Körperteile des Probanden und des Nutzers relativ zueinander zu bestimmen und den wenigstens einen dritten Stimulationsparameterwert zusätzlich basierend auf diesen Positionen und/oder Ausrichtungen und/oder Geschwindigkeiten dieser Körperteile zu bestimmen.

Das hier vorgeschlagene Verfahren kann also ferner die folgenden Schritte enthalten:
Erfassen einer Haltung und/oder Bewegung wenigstens eines Teils des Körpers des Nutzers und
Bestimmen oder Schätzen des wenigstens einen dritten Stimulationsparameterwertes zusätzlich basierend auf der erfassten Haltung und/oder Bewegung des Teils des Körpers des Nutzers.

Das System kann ferner wenigstens eine mit der Steuereinheit verbundene oder verbindbare Schnittstelle zum Empfangen wenigstens eines Fahrzeugparameterwertes wenigstens eines Fahrzeugparameters eines Fahrzeugs, z. B. eines Kraftfahrzeugs oder eines Flugzeugs, in dem das System und der Nutzer anordenbar sind, umfassen. Die Steuereinheit kann dann eingerichtet sein, den wenigstens einen dritten Stimulationsparameterwert zusätzlich basierend auf dem wenigstens einen Fahrzeugparameterwert zu bestimmen oder zu schätzen.

Das hier vorgeschlagene Verfahren kann also ferner die folgenden Schritte enthalten:
Erfassen wenigstens eines Fahrzeugparameterwertes wenigstens eines Fahrzeugparameters eines Fahrzeugs, z. B. eines Kraftfahrzeugs oder eines Flugzeugs, in dem das System und der Nutzer angeordnet sind, und
Bestimmen der Schätzen des wenigstens einen dritten Stimulationsparameterwertes zusätzlich basierend auf dem wenigstens einen Fahrzeugparameterwert.

Der wenigstens eine Fahrzeugparameter kann z. B. einen, mehrere oder alle der folgenden Parameter umfassen:
- eine Fahrzeuggeschwindigkeit,
- eine Fahrzeugbeschleunigung,
- eine Fahrzeugmasse,
- eine Fahrzeuglage,
- eine Fahrzeugreisehöhe,
- einen Strömungswiderstand des Fahrzeugs,
- einen Umgebungsluftdruck,
- eine Umgebungstemperatur,
- einen Steuerparameter zum Beeinflussen der Fahrzeuggeschwindigkeit, der Fahrzeugbeschleunigung, der Fahrzeuglage und/oder der Fahrzeugreisehöhe, wobei der Steuerparameter beispielsweise eine Position einer Steuervorrichtung zum Steuern des Fahrzeugs sein kann, z. B. die Position eines Lenkrades, eines Pedals, eines Hebels, eines Joysticks, eines Schalters oder dergleichen.

Ausführungsbeispiele der Erfindung sind in den Figuren dargestellt und werden anhand der nachfolgenden Beschreibung näher erläutert.

Es zeigt
- Fig. 1: schematisch eine Ausführungsform eines Systems zum Stimulieren wenigstens eines Muskels mittels elektrischer Stimulation;
- Fig. 2a: schematisch eine Seitenansicht eines Probanden, dessen rechter Trizeps während einer ersten Kalibrierungsphase des Systems aus Fig. 1 elektrisch stimuliert wird;
- Fig. 2b: schematisch zwei Seitenansichten des Probanden aus Fig. 2a während der ersten Kalibrierungsphase des Systems 1, wobei der Proband mit dem linken Arm ein reales Gewicht hält und eine elektrische Stimulation des rechten Trizeps im rechten Arm bei dem Probanden eine entsprechende Gewichtsempfindung hervorruft;
- Fig. 3: schematisch eine Seitenansicht eines von dem Probanden aus den Figuren 2a und 2b verschiedenen Nutzers, dessen rechter Trizeps während einer zweiten Kalibrierungsphase des Systems aus Fig. 1 elektrisch stimuliert wird;
- Fig. 4: schematisch eine Seitenansicht des Nutzers aus Fig. 3, dessen rechter Trizeps mittels des Systems aus Fig. 1 während einer Anwendung elektrisch stimuliert wird, wobei die elektrische Stimulierung auf Kalibrierungsdaten basiert, die während der ersten Kalibrierungsphase am Probanden und während der zweiten Kalibrierungsphase am Nutzer erfasst wurden;
- Fig. 5a: einen Graphen, der für eine Anzahl von Probanden jeweils Werte einer infolge elektrischer Muskelstimulation durch den jeweiligen Probanden wahrgenommenen Kraft als Funktion einer Amplitude der elektrischen Muskelstimulation darstellt und der für einen Nutzer entsprechende geschätzte Werte einer durch den Nutzer wahrgenommenen Kraft darstellt;
- Fig. 5b: einen Graphen entsprechend Fig. 5a, wobei die Schätzung der Werte des Nutzers zusätzlich auf einem bekannten Oberarmumfang des Nutzers basiert; und
- Fig. 5c: einen Graphen entsprechend Fig. 5a, wobei die Schätzung der Werte des Nutzers zusätzlich auf einem bekannten Oberarmumfang und auf einer bekannten Körpergröße des Nutzers basiert.

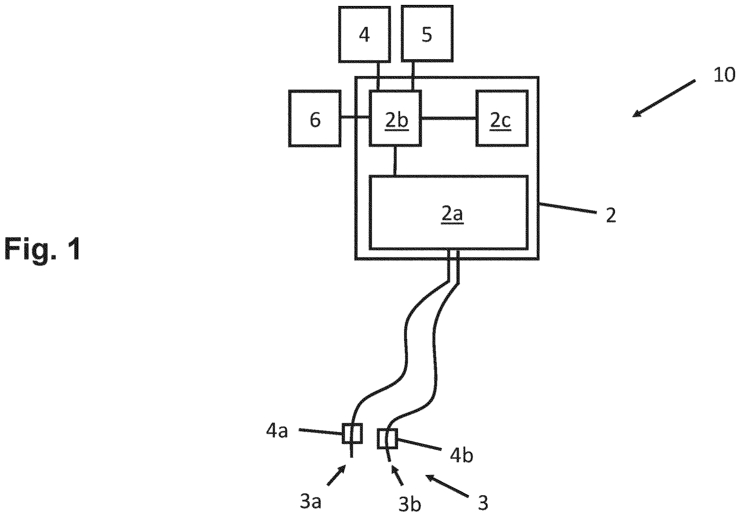

Fig. 1 zeigt schematisch ein System 10 zum Stimulieren eines oder mehrerer Muskeln mittels elektrischer Stimulation. Das System 10 umfasst eine Steuereinheit 2 und wenigstens ein Elektrodenpaar 3 mit Elektroden 3a und 3b. Das Elektrodenpaar 3 ist elektrisch mit der Steuereinheit 2 verbunden und ist über die Steuereinheit 2 ansteuerbar. Obwohl in Fig. 1 der Einfachheit halber nur ein einziges Elektrodenpaar 3 dargestellt ist, versteht es sich, dass das System 10 in anderen, hier nicht explizit dargestellten Ausführungsformen eine Vielzahl von Elektrodenpaaren 3 enthalten kann.

Die Steuereinheit 2 ist eingerichtet, an das Elektrodenpaar 3 eine elektrische Spannung anzulegen und so eine über das Elektrodenpaar 3 verabreichbare elektrische Stimulation zu erzeugen und zu kontrollieren. Eine von der Steuereinheit 2 über das wenigstens eine Elektrodenpaar 3 verabreichte elektrische Stimulation umfasst typischerweise eine Folge elektrischer Stimulationspulse. Die Steuereinheit 2 ist dann eingerichtet, einen oder mehrere der folgenden Stimulationsparameter der über das wenigstens eine Elektrodenpaar 3 verabreichten elektrischen Stimulationspulse einzustellen und/oder kontrollieren und/oder zu regeln: eine Stimulationspulsform, eine Stimulationspulsamplitude, z. B. in Form einer maximalen elektrischen Spannung oder einer maximalen elektrischen Stromstärke, eine Stimulationspulsfrequenz, eine Pulsdauer, eine Gesamtdauer der elektrischen Stimulation und/oder eine verabreichte Gesamtenergie der elektrischen Stimulation.

Die Elektroden 3a, 3b des Elektrodenpaares 3 sind dazu ausgebildet, einen oder mehrere Muskeln eines Menschen oder eines Tieres durch die Haut hindurch elektrisch zu stimulieren. Das System 10 ist also besonders zur Stimulation von Muskeln geeignet, die nahe unter der Haut des Körpers liegen und durch die Haut hindurch stimulierbar sind. Insbesondere sind die Elektroden 3a, 3b des Elektrodenpaares 3 dazu ausgebildet, derart auf der Haut eines Menschen oder eines Tieres befestigt zu werden, dass sie in Kontakt mit der Haut sind. Zu diesem Zweck kann das System 10 z. B. Befestigungsmittel 4a, 4b zum Befestigen der Elektroden 3a, 3b auf der Haut eines Menschen oder eines Tieres aufweisen. Die Befestigungsmittel 4a, 4b können z. B. jeweils eine Manschette mit einstellbarem Umfang, ein Klebeband, einen Saugnapf oder dergleichen beinhalten.

Die Steuereinheit 2 umfasst z. B. einen mit dem Elektrodenpaar 3 elektrisch verbundenen oder verbindbaren elektrischen Pulsgenerator 2a zum Erzeugen und/oder Kontrollieren und/oder Regeln der elektrischen Stimulation, eine mit dem Pulsgenerator 2a elektrisch verbundene oder verbindbare Kontrolleinheit 2b zum Einstellen von Stimulationsparameterwerten der Stimulationsparameter der elektrischen Stimulation, z. B. in Gestalt eines programmierbaren Mikrocontrollers oder eines programmierbaren Mikroprozessors, und eine mit der Kontrolleinheit 2b elektrisch verbundene oder verbindbare Datenspeichereinheit 2c.

Das System 10 kann ferner eines oder mehrere der folgenden mit der Steuereinheit 2 bzw. mit der Kontrolleinheit 2a verbundenen oder verbindbaren Komponenten aufweisen: eine Eingabeeinheit 4, z. B. in Form einer Tastatur, eines Touchscreens oder eines Mikrophons in Verbindung mit einer Spracherkennungssoftware, ein Bewegungs-Erfassungssystem 5, z. B. in Form wenigstens einer Kamera oder eines oder mehrerer inertialer Messeinheiten, eine Schnittstelle 6 zum Empfangen eines oder mehrerer Fahrzeugparameterwerte. Die Funktion der Komponenten 4, 5, 6 wird an späterer Stelle erläutert.

Wie im Weiteren näher erläutert wird, zeichnet sich das System 10 besonders dadurch aus, dass die Steuereinheit 2 derart eingerichtet und/oder programmiert ist, dass bei einer Anwendung des Systems 10 ein gewünschtes Ergebnis einer über das Elektrodenpaar 3 verabreichten elektrischen Stimulation eines oder mehrerer Muskeln eines Nutzers für eine Vielzahl verschiedener Nutzer jeweils mit geringem Kalibrierungsaufwand und gleichzeitig guter Genauigkeit erzielt werden kann. Abhängig von der jeweiligen Anwendung kann das gewünschte Ergebnis der über das Elektrodenpaar 3 verabreichten Stimulation z. B. eine infolge der Stimulation beim Nutzer hervorgerufene Kraftempfindung einer gewünschten Größe und/oder eine infolge der Stimulation durch den einen oder die mehreren stimulierten Muskeln des Nutzers erzeugte Kraft einer gewünschten Größe sein.

Um dies zu erreichen, muss das System 10 ebenso wie aus dem Stand der Technik bekannte verwandte Systeme kalibriert werden.

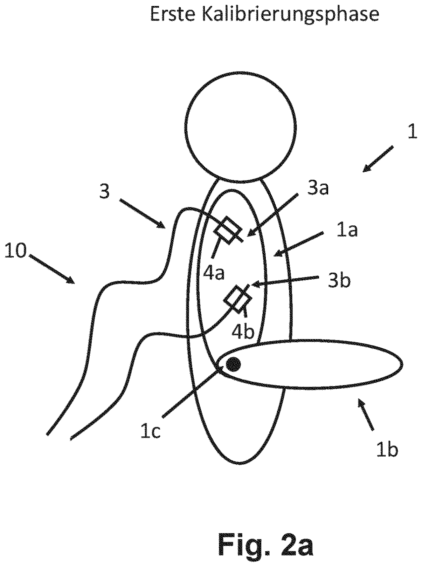

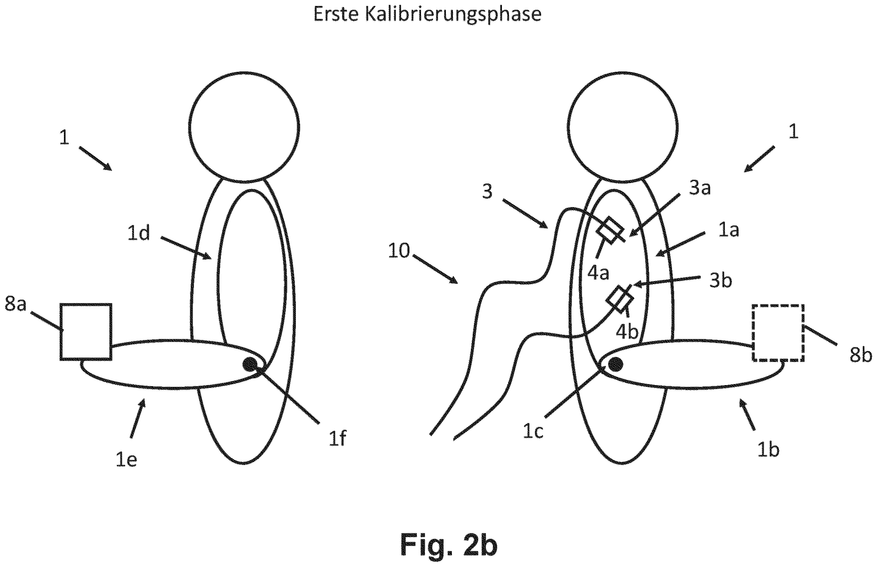

Die Figuren 2a und 2b zeigen jeweils schematisch eine Situation einer ersten Kalibrierungsphase zur Kalibrierung des Systems 10 an einem Probanden 1. Dabei sind hier und im Folgenden in verschiedenen Figuren wiederkehrende Merkmale jeweils mit denselben Bezugszeichen bezeichnet.

Wie im Folgenden näher erläutert wird, wird in der in Fig. 2a dargestellten Situation der ersten Kalibrierungsphase für den Probanden 1 zunächst eine untere und ein obere Grenze für Werte eines Stimulationsparameters bestimmt und in der Datenspeichereinheit 2c der Steuereinheit 2 gespeichert. Daraufhin wird in der in Fig. 2b dargestellten Situation der ersten Kalibrierungsphase für verschiedene innerhalb der zuvor bestimmten Grenzen gelegene Werte desselben Stimulationsparameters jeweils eine Kraftempfindung des Probanden 1 erfasst, wobei der Wert des Stimulationsparameters und die ihm zugeordnete Kraftempfindung des Probanden 1 in der Datenspeichereinheit 2c der Steuereinheit 2 gespeichert werden.

Wie Fig. 2a zu entnehmen ist, hat der Proband 1 einen rechten Oberarm 1a, einen rechten Unterarm 1b und einen rechten Ellenbogen 1c. Im Bereich der Enden des rechten Trizeps des Probanden 1 sind auf der Haut des Probanden 1 mithilfe der Befestigungsmittel 4a, 4b die Elektroden 3a, 3b des Elektrodenpaares 3 des Systems 10 befestigt, siehe Fig. 1, und sind mit der Haut des Probanden 1 in Kontakt. Der rechte Oberarm 1a liegt in vertikaler Richtung seitlich am Rumpf an. Gleichzeitig hält der Proband 1 seinen rechten Unterarm 1b nach vorne und beugt ihn derart, dass der rechte Oberarm 1a und der rechte Unterarm 1b einen Winkel von 90 Grad einschließen.

In der in Fig. 2a gezeigten Situation steuert die Steuereinheit 2 die Elektroden 3a, 3b des Elektrodenpaares 3 derart an, dass der rechte Trizeps des Probanden 1 über das Elektrodenpaar 3 elektrisch stimuliert wird. Abhängig von der Intensität der elektrischen Stimulation wird der rechte Trizeps des Probanden 1 zu einer Kontraktion veranlasst, die in Bezug auf den rechten Ellenbogen 1c des Probanden ein Drehmoment erzeugt und den rechten Unterarm 1b des Probanden dazu veranlasst, sich zu strecken und den Winkel zwischen dem rechten Oberarm 1a und dem rechten Unterarm 1b zu vergrößern. Der Proband 1 ist jedoch angehalten, den rechten Unterarm 1b in der in Fig. 2a gezeigten Position zu halten. Dazu muss der Proband 1 eine von der Intensität der elektrischen Stimulation des rechten Trizeps abhängige Kraft mit dem rechten Bizeps, dem Antagonisten des Trizeps, aufwenden, um das durch den stimulierten rechten Trizeps in Bezug auf den rechten Ellenbogen 1c erzeugte Drehmoment zu kompensieren. Der Proband 1 nimmt dabei im rechten Bizeps eine Kraft wahr, die einer Kraftwahrnehmung beim Halten eines realen Gewichts mit dem rechten Unterarm entspricht.

In der in Fig. 2a und 2b gezeigten Situation erzeugt der Pulsgenerator 2a der Steuereinheit 2 zum elektrischen Stimulieren des rechten Trizeps des Probanden 1 eine Folge elektrischer Stimulationspulse, die dem rechten Trizeps des Probanden 1 über das Elektrodenpaar 3 verabreicht werden. Die von der Steuereinheit 2 erzeugten elektrischen Stimulationspulse sind durch Stimulationsparameter wie eine Stimulationspulsform, eine Stimulationspulsamplitude (gemessen in mA), eine Stimulationspulsfrequenz, eine Pulsdauer, eine Gesamtdauer der elektrischen Stimulation und eine Gesamtenergie der elektrischen Stimulation charakterisiert. Für die in Fig. 2a und Fig. 2b dargestellte erste Kalibrierungsphase können die Werte der o. g. Stimulationsparameter z. B. manuell über die Eingabeeinheit 4 des Systems 10 eingegeben werden.

Im vorliegenden Beispiel werden in der in Fig. 2a und Fig. 2b dargestellten ersten Kalibrierungsphase jeweils die folgenden Stimulationsparameterwerte verwendet:

| | |
|---|---|
| - Stimulationspulsform: | Rechteckpulse alternierender Polarität, |
| - Stimulationspulsrequenz: | 120 Hz, |
| - Stimulationspulsbreite: | 80 µs, |
| - Stimulationsdauer: | 1s. |

In Fig. 2a wird die so charakterisierte elektrische Stimulation des rechten Trizeps des Probanden 1 mehrfach wiederholt, wobei die Steuereinheit 2 für jede der Wiederholungen z. B. automatisch einen anderen Wert der Stimulationsamplitude einstellt. Die Steuereinheit 2 erhöht die Stimulationspulsamplitude beginnend bei einem Wert von z. B. 5 mA schrittweise von Wiederholung zu Wiederholung, z. B. in Schritten von 5 mA oder von 10 mA. Zwischen den Wiederholungen mit schrittweise erhöhter Stimulationspulsamplitude kann jeweils eine Pause von z. B. 10 s oder 20 s vorgesehen sein, während derer die elektrische Stimulation unterbrochen wird. Dieser Vorgang wird abgebrochen, sobald die Stimulationspulsamplitude einen Wert erreicht, bei dem der Proband 1 infolge der elektrischen Stimulation das Einsetzen eines geringfügigen Unwohlseins verspürt oder der einen zulässigen Höchstwert überschreitet.

Während der Durchführung dieser Reihe von Wiederholungen an dem Probanden 1 mit zunehmender Stimulationspulsamplitude werden für den Probanden 1 zwei Werte der Stimulationspulsamplitude registriert und z. B. über die Eingabeeinheit 4 des Systems 10 eingegeben und in der Datenspeichereinheit 2c gespeichert:
- der motorische Schwellenwert (im Folgenden auch: MS) und
- die Grenze des Unwohlseins (im Folgenden auch: GU).

Dabei ist der motorische Schwellenwert oder MS der kleinste Wert der Stimulationspulsamplitude, der den rechten Trizeps des Probanden 1 noch zu einer Kontraktion veranlasst, und die Grenze des Unwohlseins oder GU ist der größte Wert der Stimulationspulsamplitude, der den rechten Trizeps des Probanden 1 noch ohne ein geringfügiges Unwohlsein des Probanden 1 infolge der elektrischen Stimulation zu einer Kontraktion veranlasst. Für den Probanden 1 und die oben aufgeführten übrigen Stimulationsparameterwerte beträgt der Wert des motorischen Schwellenwerts der Stimulationspulsamplitude MS = 14 mA, und der Wert der Grenze des Unwohlseins der Stimulationspulsamplitude beträgt GU = 46 mA.

In Fig. 2b ist der Proband 1 einmal in einer Seitenansicht von links und einmal in einer Seitenansicht von rechts dargestellt. Gezeigt sind insbesondere der rechte Oberarm 1a, der rechte Unterarm 1b, der rechte Ellenbogen 1c, der linke Oberarm 1d, der linke Unterarm 1e und der linke Ellenbogen 1f des Probanden 1. Die Oberarme 1a, 1d liegen jeweils in vertikaler Richtung seitlich am Rump des Probanden 1 an. Gleichzeitig hält der Proband 1 seine Unterarme 1b, 1e nach vorne und beugt sie derart, dass sie mit den Oberarmen 1a, 1d jeweils einen Winkel von 90 Grad einschließen.

In Fig. 2b hält der Proband 1 nun mit seinem linken Unterarm 1e ein reales Gewicht 8a einer bekannten Masse von z. B. 1 kg. Um das reale Gewicht 8a statisch in dieser in Fig. 2b dargestellten Position zu halten, muss der Proband 1 mit seinem linken Bizeps eine Kraft aufwenden, die in Bezug auf seinen linken Ellenbogen 1f ein Drehmoment erzeugt, das dem durch die Gewichtskraft des realen Gewichts 8a in Bezug auf seinen linken Ellenbogen 1f erzeugten Drehmoment entgegenwirkt und dieses kompensiert. Dies ruft bei dem Probanden 1 in seinem linken Bizeps wieder eine Kraftempfindung hervor, deren Größe von der Größe des Drehmoments abhängt, das durch die Gewichtskraft der bekannten Masse des realen Gewichts 8a erzeugt wird. Außer von der Masse des realen Gewichts 8a hängt das Drehmoment, das von der Gewichtskraft des realen Gewichts 8a in Bezug auf den linken Ellenbogen 1f des Probanden 1 erzeugt wird, in bekannter Weise von der Länge des linken Unterarms 1e des Probanden 1 und von dem Beugungswinkel zwischen dem linken Oberarm 1d und dem linken Unterarm 1e des Probanden 1 ab.

Während der Proband 1 mit dem linken Unterarm 1e das reale Gewicht 8a der Masse 1 kg hält, wird der rechte Trizeps des Probanden 1 wie bereits in Bezug auf Fig. 2a beschrieben über die Elektroden 3a, 3b des Elektrodenpaars 3 elektrisch stimuliert, wobei der Proband 1 wie zuvor angehalten ist, seinen rechten Unterarm 1b in der in Fig. 2b gezeigten Position zu halten. In Fig. 2b werden für die Stimulationsparameter Stimulationspulsform, Stimulationspulsfrequenz, Stimulationspulsbreite und Stimulationsdauer die zuvor angegebenen Stimulationsparameterwerte verwendet. Wie zuvor in Bezug auf Fig. 2a beschrieben nimmt der Proband 1 infolge der elektrischen Stimulation seines rechten Trizeps und des Haltens des rechten Unterarms 1b in der in Fig. 2b gezeigten Position in seinem rechten Bizeps eine Kraft wahr, die der Kraftwahrnehmung beim Halten eines realen Gewichts mit dem rechten Unterarm 1b entspricht. Diese Kraftwahrnehmung des Probanden 1 im rechten Bizeps infolge der elektrischen Stimulation ist in Fig. 2b als ein scheinbares oder virtuelles Gewicht 8b dargestellt.

Die Steuereinheit 2 variiert den Wert der Stimulationspulsamplitude nun, bis der Proband 1 signalisiert, dass seine Kraftempfindung im rechten Bizeps infolge der elektrischen Stimulation seines rechten Trizeps seiner Kraftempfindung im linken Bizeps infolge des Haltens des realen Gewichts 8a der Masse 1 kg mit seinem linken Unterarm 1e entspricht. Dabei darf der zuvor für den Probanden 1 aufgefundene Wert der Stimulationspulsamplitude von GU = 46 mA nicht überschritten werden.

Der Wert der Stimulationspulsamplitude, der in der in Fig. 2b dargestellten Situation im rechten Bizeps des Probanden 1 dieselbe Kraftempfindung hervorruft wie das Halten des realen Gewichts 8a mit dem linken Unterarm 1e, und der Wert der zugehörigen Gewichtskraft können dann z. B. über die Eingabeeinheit 4 des Systems 10 eingegeben und in der Datenspeichereinheit 2c gespeichert werden.

Das in Bezug auf Fig. 2b beschriebene Vorgehen kann mit einer Vielzahl von Gewichten unterschiedlicher Masse wiederholt werden, typischerweise mit Gewichten mit Massen zwischen 0,5 kg und ca. 8 kg. Auf diese Weise kann die Steuereinheit 2 während der in den Figuren 2a und 2b dargestellten ersten Kalibrierungsphase für den ersten Probanden 1 neben den Werten MS und GU der Stimulationspulsamplitude eine Vielzahl von Werten der Stimulationspulsamplitude (in mA) und einen diesen Werten der Stimulationspulsamplitude jeweils zugeordneten Wert einer Kraftempfindung (in N) als physischen Parameterwert des Probanden 1 speichern.

Um den Probanden 1 noch weitergehend zu charakterisieren, können während der am Probanden 1 durchgeführten ersten Kalibrierungsphase des Systems 10 über die Eingabeeinheit 4 zusätzlich Werte weiterer physischer Parameter des Probanden 1 eingegeben und in der Datenspeichereinheit 2c gespeichert werden. Im hier beschriebenen Beispiel werden als weitere physische Parameterwerte des Probanden 1 die Körpergröße (im Folgenden auch: KG) des Probanden 1 und der maximale Umfang des rechten Oberarms 1a (im Folgenden auch: OU) des Probanden 1 über die Eingabeeinheit 4 eingegeben und in der Datenspeichereinheit 2c gespeichert, nämlich KG = 155 cm und OU = 22 cm.

In alternativen, hier nicht explizit beschriebenen Ausführungsformen können während der am Probanden 1 durchgeführten ersten Kalibrierungsphase des Systems 10 als weitere physische Parameter des Probanden 1 zusätzlich einer oder mehrere der folgenden Parameter über die Eingabeeinheit 4 eingegeben und in der Datenspeichereinheit 2c gespeichert werden:
- eine Bezeichnung eines während der ersten Kalibrierungsphase stimulierten Muskels des Probanden 1,
- das Körpergewicht des Probanden 1,
- das Lebensalter des Probanden 1,
- das Geschlecht des Probanden 1,
- der Körperfettanteil des Probanden 1,
- die Knochenmasse des Probanden 1,
- der Körperflüssigkeitsanteil des Probanden 1,
- die Muskelmasse des Probanden 1,
- ein Größenmaß wenigstens eines weiteren Körperteils des Probanden 1,
- eine maximal generierbare Kraft durch willentliche Aktivierung eines Muskels des Probanden 1, z. B. seines rechten Trizeps,
- infolge der elektrischen Stimulation während der ersten Kalibrierungsphase durch den rechten Trizeps des Probanden 1 erzeugte Kraftwerte, messbar z. B. mittels eines Kraftsensors.

Typischerweise wird die hier in Bezug auf die Figuren 2a und 2b explizit nur für den Probanden 1 beschriebene erste Kalibrierungsphase in entsprechender Weise zusätzlich für eine Vielzahl vom Probanden 1 verschiedener weiterer Probanden durchgeführt, z. B. für insgesamt wenigstens 5 Probanden, für insgesamt wenigstens 10 Probanden, für insgesamt wenigstens 20 Probanden oder für insgesamt wenigstens 50 Probanden.

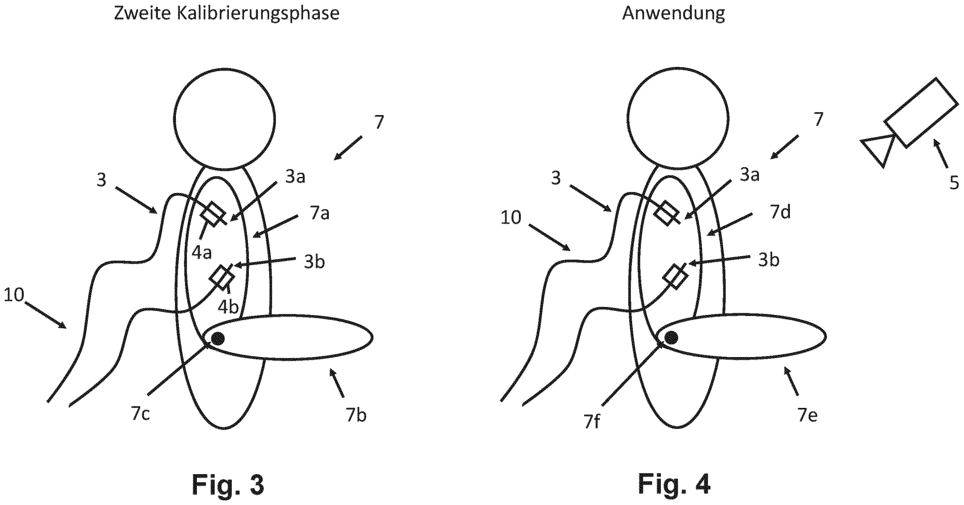

Fig. 3 zeigt schematisch eine Situation einer zweiten Kalibrierungsphase zur Kalibrierung des Systems 10 an einem von dem Probanden 1 verschiedenen Nutzer 7.

Wie Fig. 3 zu entnehmen ist, hat der Nutzer 7 einen rechten Oberarm 7a, einen rechten Unterarm 7b und einen rechten Ellenbogen 7c. Im Bereich der Enden des rechten Trizeps des Nutzers 7 sind auf der Haut des Nutzers 7 mithilfe der Befestigungsmittel 4a, 4b die Elektroden 3a, 3b des Elektrodenpaares 3 des Systems 10 befestigt, siehe Fig. 1, und sind mit der Haut des Nutzers 7 in Kontakt. Der rechte Oberarm 7a liegt in vertikaler Richtung seitlich am Rumpf an. Gleichzeitig hält der Nutzer 7 seinen rechten Unterarm 7b nach vorne und beugt ihn derart, dass der rechte Oberarm 7a und der rechte Unterarm 7b einen Winkel von 90 Grad einschließen.

Entsprechend dem in Bezug auf Fig. 2a beschriebenen Vorgehen erzeugt der Pulsgenerator 2a der Steuereinheit 2 während der in Fig. 3 dargestellten zweiten Kalibrierungsphase zum elektrischen Stimulieren des rechten Trizeps des Nutzers 7 eine Folge elektrischer Stimulationspulse, die dem rechten Trizeps des Nutzers 7 über das Elektrodenpaar 3 verabreicht werden, wobei während der am Nutzer 7 durchgeführten zweiten Kalibrierungsphase die Werte der Stimulationsparameter Stimulationspulsform, Stimulationspulsfrequenz, Stimulationspulsbreite und Stimulationsdauer entsprechend den Werten dieser Stimultionsparameter während der am Probanden 1 durchgeführten ersten Kalibrierungsphase gewählt werden:

| | |
|---|---|
| - Stimulationspulsform: | Rechteckpulse alternierender Polarität, |
| - Stimulationspulsrequenz: | 120 Hz, |
| - Stimulationspulsbreite: | 80 µs, |
| - Stimulationsdauer: | 1 s. |

Ganz entsprechend dem oben in Bezug auf Fig. 2a beschriebenen Vorgehen werden während der am Nutzer 7 durchgeführten und in Fig. 3 dargestellten zweiten Kalibrierungsphase nun für den Nutzer 7 der motorische Schwellenwert MS und die Grenze des Unwohlseins GU der Stimulationspulsamplitude bestimmt, über die Eingabeeinheit 4 eingegeben und in der Datenspeichereinheit 2c der Steuereinheit 2 gespeichert. Ferner werden im hier gezeigten Beispiel während der zweiten Kalibrierungsphase über die Eingabeeinheit 4 der Wert des Körpergewichts KG des Nutzers 7 und der Wert des maximalen Umfangs des rechten Oberarms 7a OU des Nutzers 7 als physische Parameterwerte des Nutzers 7 eingegeben und in der Datenspeichereinheit 2c der Steuereinheit 2 gespeichert.

Um den Nutzer 7 noch weitergehend zu charakterisieren, können während der am Nutzer 7 durchgeführten zweiten Kalibrierungsphase des Systems 10 über die Eingabeeinheit 4 zusätzlich Werte weiterer physischer Parameter des Nutzers 7 eingegeben und in der Datenspeichereinheit 2c gespeichert werden. Im hier beschriebenen Beispiel werden als weitere physische Parameterwerte des Nutzers 7 die Körpergröße (im Folgenden auch: KG) des Nutzers 7 und der maximale Umfang des rechten Oberarms 7a (im Folgenden auch: OU) des Nutzers 7 über die Eingabeeinheit 4 eingegeben und in der Datenspeichereinheit 2c gespeichert, nämlich KG = 200 cm und OU = 30 cm.

In alternativen, hier nicht explizit beschriebenen Ausführungsformen können während der am Nutzer 7 durchgeführten zweiten Kalibrierungsphase des Systems 10 als weitere physische Parameter des Nutzers 7 zusätzlich einer oder mehrere der folgenden Parameter über die Eingabeeinheit 4 eingegeben und in der Datenspeichereinheit 2c gespeichert werden:
- eine Bezeichnung eines während der zweiten Kalibrierungsphase stimulierten Muskels des Nutzers 7,
- das Körpergewicht des Nutzers 7,
- das Lebensalter des Nutzers 7,
- das Geschlecht des Nutzers 7,
- der Körperfettanteil des Nutzers 7,
- die Knochenmasse des Nutzers 7,
- der Körperflüssigkeitsanteil des Nutzers 7,
- die Muskelmasse des Nutzers 7,
- ein Größenmaß wenigstens eines weiteren Körperteils des Nutzers 7,
- eine maximal generierbare Kraft durch willentliche Aktivierung eines Muskels des Nutzers 7, z. B. seines rechten Trizeps,
- infolge der elektrischen Stimulation während der zweiten Kalibrierungsphase durch den rechten Trizeps des Nutzers 7 erzeugte Kraftwerte, messbar z. B. mittels eines Kraftsensors.

Fig. 4 zeigt schematisch eine Seitenansicht des Nutzers 7 aus Fig. 3 bei einer Anwendung des Systems 10. Bei der in Fig. 4 dargestellten Anwendung sind im Bereich der Enden des rechten Trizeps des Nutzers 7 auf der Haut des Nutzers 7 mithilfe der Befestigungsmittel 4a, 4b die Elektroden 3a, 3b des Elektrodenpaares 3 des Systems 10 befestigt, siehe Fig. 1, und sind mit der Haut des Nutzers 7 in Kontakt. Während der Anwendung des Systems 10 soll mit der Stimulierung des rechten Trizeps des Nutzers 7 ein gewünschtes Ergebnis erzielt werden, z. B. eine Kraftempfindung einer vorgegebenen Größe durch den Nutzer 7.

Fig. 4 zeigt ferner das mit der Steuereinheit 2 verbundene oder verbindbare Bewegungs-Erfassungssystem 5 des Systems 10, hier in Gestalt einer Kamera, z. B. einer Stereokamera. Mithilfe des Bewegungs-Erfassungssystems 5 kann eine Haltung und/oder Bewegung des Nutzers 7 oder wenigstens eines oder mehrerer Körperteile des Nutzers 7 in Echtzeit erfasst werden. Beispielsweise kann die Steuereinheit 2 eingerichtet sein, anhand von mittels des Bewegungs-Erfassungssystems 5 aufgenommenen Bilddaten in Echtzeit den vom Oberarm 7a und vom Unterarm 7b des Nutzers 7 eingeschlossenen Winkel zu bestimmen oder zu schätzen. Die Steuereinheit 2 kann dann ferner eingerichtet sein, einen oder mehrere Stimulationsparameterwerte, die die elektrische Stimulation des Trizeps des Nutzers 7 charakterisieren und mit denen eine gewünschte muskuläre Antwort erzielt werden soll, beispielsweise eine Kraftempfindung einer vorgegebenen Größe, ferner basierend auf dem so bestimmten Wert des vom Oberarm 7a und vom Unterarm 7b des Nutzers 7 eingeschlossenen Winkels zu bestimmen oder zu schätzen.

In einem ersten Beispiel kann der Nutzer 7 sich während der in Fig. 4 gezeigten Anwendung in einer virtuellen Realität (VR) befinden, und es soll ihm durch elektrische Stimulierung seines rechten Trizeps die Wahrnehmung einer Gewichtskraft eines virtuellen Objekts vermittelt werden, das er in der VR mit dem rechten Unterarm 1b hält und das ihm z. B. durch eine VR-Brille gezeigt wird. Zur Bestimmung oder Schätzung des Stimulationsparameterwertes, mit dem bei dem Nutzer 7 die Gewichtskraftwahrnehmung der gewünschten Größe erzielt werden soll, kann die Steuereinheit 2 z. B. in Echtzeit den Wert des Winkels zwischen dem Oberarm 7a und dem Unterarm 7b des Nutzers 7 berücksichtigen.

In einem zweiten Beispiel kann der Nutzer 7 im Sitz eines Fahrzeugs sitzen, z. B. im Pilotensitz eines Düsenjets, der im Begriff ist, ein Flugmaneuver auszuführen, bei dem der rechte Unterarm 7b des Nutzers 7 Gefahr läuft, infolge einer plötzlichen starken Beschleunigung verletzt zu werden. In diesem Fall kann es vorteilhaft sein, einer infolge des bevorstehenden Flugmaneuvers erwarteten Krafteinwirkung auf den Unterarm 7b des Nutzers 7 bereits unmittelbar bei Beginn des Flugmaneuvers durch elektrische Stimulierung des rechten Trizeps des Nutzers 7 entgegenzuwirken. Ist das bevorstehende Flugmaneuver beispielsweise ein plötzlicher steiler Sinkflug, so könnte der rechte Unterarm 7b des Nutzers 7 infolge der bei diesem Flugmaneuver wirkenden Trägheitskräfte plötzlich nach oben in Richtung auf die Schulter des Nutzeres 7 schnellen. Dies könnte z. B. eine Verletzung des rechten Unterarms 7b und/oder die Beschädigung von Gegenständen im Cockpit zur Folge haben. In dieser Situation könnte der rechte Trizeps des Nutzers 7 unmittelbar zu Beginn des Sinkflugs durch gezielte elektrische Stimulation veranlasst werden, den rechten Unterarm 7b des Nutzers 7 von der rechten Schulter des Nutzers 7 weg nach unten zu drücken, beispielsweise gegen eine Unterarmstütze, um den zu Beginn des steilen Sinkflugs auf den Unterarm 7b des Nutzeres 7 einwirkenden Trägkeitskräften entgegenzuwirken. Im Beispiel des Jetpiloten kann es z. B. auch besonders vorteilhaft sein, die Nackenmuskulatur des Jetpiloten kurz vor einem Flugmaneuver mit starker Beschleunigung zu stimulieren, um einem plötzlichen Umknicken des Kopfes des Piloten infolge des Flugmaneuvers entgegenzuwirken.

Um eine auf den Jetpiloten oder auf einzelne Körperteile des Jetpiloten einwirkende Kraft bei einem bevorstehenden Flugmaneuver abschätzen und bei der Bestimmung oder Schätzung eines oder mehrerer Stimulationsparameterwerte, die die elektrische Stimulation einer oder mehrerer Muskeln des Jetpiloten charakterisieren, berücksichtigen zu können, kann die Steuereinheit 2 über die Schnittstelle 6, siehe Fig. 1, Flugzeugparameterwerte eines oder mehrerer Flugzeugparameter empfangen, die einen Einfluss auf die Kräfte haben können, die infolge des Flugmaneuvers auf den Jetpiloten oder einzelne seiner Körperteile einwirken können. Diese Flugzeugparameter können beispielsweise die Stellung eines Joysticks zum Steuern des Düsenjets umfassen. Dies kann besonders vorteilhaft sein, da eine Veränderung der Stellung des Joysticks aufgrund der Trägheit des Düsenjets ein durch die Stellungsänderung des Joysticks ausgelöstes Flugmaneuer, z. B. einen plötzlichen steilen Sinkflug oder dergleichen, gewöhnlich mit einer gewissen zeitlichen Verzögerung vorwegnimmt. Weitere Flugzeugparameter, die die Steuereinheit 2 über die Schnittstelle 6 empfangen und bei der Bestimmung oder Schätzung des oder der Stimulationsparameterwerte berücksichtigen können, können wenigstens einen oder mehrere der folgenden Parameter umfassen:
- eine aktuelle Geschwindigkeit des Düsenjets,
- eine aktuelle Beschleunigung des Düsenjets,
- eine Masse des Düsenjets,
- eine aktuelle Lage des Düsenjets, z. B. Roll-, Nick- und Gierwinkel,
- aktuelle Flughöhe des Düsenjets,
- einen Strömungswiderstand des Düsenjets,
- einen Umgebungsluftdruck,
- eine Umgebungstemperatur.

In einem dritten Beispiel kann der Nutzer 7 ein Sportler sein, der einen Bewegungsablauf einstudieren möchte, z. B. eine Bewegung seines rechten Oberarms 7a und seines rechten Unterarms 7b beim Aufschlag im Tennis oder den Schwung beim Golf. In diesem Beispiel kann die Bewegungs-Erfassungseinheit 5 den Bewegungsablauf des Nutzers 7 in Echtzeit erfassen, mit einem in der Datenspeichereinheit 2c gespeicherten idealen Bewegungsablauf vergleichen und die Bewegung des Nutzers 7 durch elektrische Stimulation korrigieren, wenn der mittels der Bewegungs-Erfassungseinheit 5 erfasste Bewegungsablauf des Nutzers 7 um mehr als eine vorgegebene Toleranz von dem idealen Bewegungsablauf abweicht.

In all diesen Anwendungsbeispielen soll durch die von dem System 10 verabreichte elektrische Stimulation eine Kraftempfindung einer vorgegebenen Größe (in N) mit möglichst guter Genauigkeit hervorgerufen werden. Hierzu muss die Steuereinheit 2 eingerichtet sein, in Echtzeit diejenigen Stimulationsparameterwerte zu bestimmen oder zu schätzen, mit denen die gewünschte muskuläre Antwort erzielt werden kann, und den zu stimulierenden Muskel über das Elektrodenpaar 3 mit einer durch diese Stimulationsparameterwerte charakterisierten Stimulationspulsfolge zu beaufschlagen.

Entsprechend dem in Bezug auf Fig. 3 beschriebenen Vorgehen während der zweiten Kalibrierungsphase erzeugt der Pulsgenerator 2a der Steuereinheit 2 während der in Fig. 4 dargestellten Anwendung zum elektrischen Stimulieren des rechten Trizeps des Nutzers 7 eine Folge elektrischer Stimulationspulse, die dem rechten Trizeps des Nutzers 7 über das Elektrodenpaar 3 verabreicht werden, wobei während der am Nutzer 7 durchgeführten Anwendung die Werte der Stimulationsparameter Stimulationspulsform, Stimulationspulsfrequenz, Stimulationspulsbreite und Stimulationsdauer entsprechend den Werten dieser Stimultionsparameter während der zuvor am Nutzer 7 durchgeführten zweiten Kalibrierungsphase gewählt werden:

| | |
|---|---|
| - Stimulationspulsform: | Rechteckpulse alternierender Polarität, |
| - Stimulationspulsrequenz: | 120 Hz, |
| - Stimulationspulsbreite: | 80 µs, |
| - Stimulationsdauer: | 1 s. |

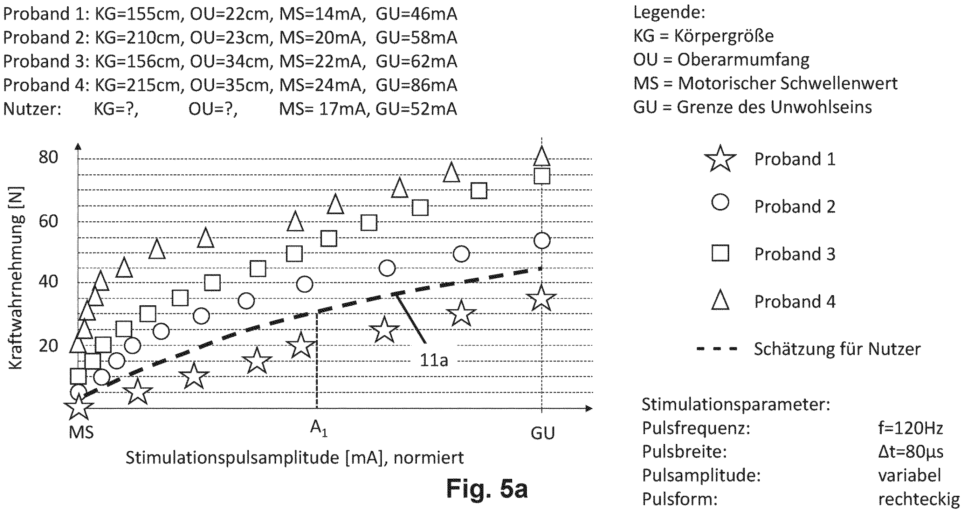

Um beim Nutzer 7 während der in Fig. 4 dargestellten Anwendung die gewünschte oder erforderliche Kraftempfindung zu erzielen, ist die Steuereinheit 2 eingerichtet, den Wert des Stimulationparameters Stimulationspulsamplitude basierend auf während der ersten Kalibrierungsphase am Probanden 1 und basierend auf während der zweiten Kalibrierungsphase am Nutzer 7 erfassten und in der Datenspeichereinheit 2c gespeicherten Kalibrierungsdaten zu bestimmen oder zu schätzen. Das Bestimmen oder Schätzen des Wertes der Stimulationspulsamplitude, mit dem beim Nutzer 7 während der Anwendung eine Kraftempfindung einer gewünschten Größe erzielt werden kann, basierend auf entsprechenden Kalibrierungsdaten ist in den Figuren 5a-c dargestellt. Beispielsweise soll die Steuereinheit 2 in der in Fig. 4 dargestellten Anwendungssituation durch elektrische Stimulation des Trizeps des Nutzers 7 beim Nutzer 7 eine Kraftwahrnehmung von 30 N hervorrufen, was z. B. einer Gewichtskraft von ca. 3 kg entspricht.

Die Figuren 5a-c zeigen jeweils einen Graphen, der während der ersten Kalibrierungsphase des Systems 10 an vier verschiedenen Probanden, darunter der Proband 1 aus den Figuren 2a und 2b, und während der zweiten Kalibrierungsphase des Systems 10 am Nutzer 7 erfasste Kalibrierungsdaten darstellt. Die in den Graphen der Figuren 5a-c dargestellten Kalibrierungsdaten wurden entsprechend der in Bezug auf die Figuren 2a und 2b beispielhaft für den Probanden 1 erläuterten Vorgehensweise erfasst und sind in der Datenspeichereinheit 2c der Steuereinheit 2 gespeichert. In den Graphen der Figuren 5a-c sind für jeden dieser vier Probanden als physische Parameterwerte Werte der Kraftwahrnehmung im rechten Bizeps, die während der ersten Kalibrierungsphase des Systems 10 bestimmt wurden, gegen die diesen Kraftwahrnehmungswerten zugeordneten Werte der Stimulationspulsamplitude aufgetragen.

Zusätzlich enthält jeder der Graphen der Figuren 5a-c für jeden der vier Probanden und für den Nutzer 7 den motorischen Schwellenwert (MS) und die Grenze des Unwohlseins (GU) der Stimulationspulsamplitude. Dabei ist in demselben Graphen für jeden der vier Probanden und für den Nutzer 7 jeweils das Intervall der Stimulationsamplitude dargestellt, das sich von MS bis GU erstreckt, wobei MS und GU für jeden der vier Probanden und den Nutzer 7 unterschiedliche absolute Werte annehmen. Die Graphen der Figuren 5a-c enthalten daher entlang der x-Achse für jeden der vier Probanden und den Nutzer 7 in Bezug auf die Absolutwerte der Stimulationspulsamplitude jeweils unterschiedliche Skalen. Zusätzlich sind in den Figuren 5a-c für jeden der vier Probanden während der ersten Kalibrierungsphase erfasste physische Parameterwerte angegeben, nämlich jeweils das Körpergewicht KG und der Umfang des rechten Oberarms OU.

Im in Fig. 5a gezeigten Beispiel bestimmt oder schätzt die Steuereinheit 2 den gesuchten Wert der Stimulationspulsamplitude, der beim Nutzer 7 eine gewünschte Kraftwahrnehmung von 30 N hervorruft, basierend auf den in Fig. 5a enthaltenen Kalibrierungsdaten der Probanden 1 bis 4 und basierend nur auf den Werten von MS und GU des Nutzers 7. Da der Steuereinheit 2 für den Nutzer 7 nur die zwei Werte von MS und GU vorliegen, wählt die Steuereinheit 2 zur Bestimmung oder Schätzung des gesuchten Wertes der Stimulationspulsamplitude aus den Kalibrierungsdatensätzen der vier Probanden nur die Kalibrierungsdatensätze derjenigen zwei Probanden aus, die dem Kalibrierungsdatensatz des Nutzers 7 im durch den physischen Parameter MS und durch den physischen Parameter GU aufgespannten zweidimensionalen Parameterraum am nächsten liegen, wobei die Steuereinheit 2 als Abstandsmaß im MS-GU-Raum die euklidische Metrik verwendet. Die zwei Probanden, deren Datensätze im MS-GU-Raum dem Datensatz des Nutzers 7 am nächsten liegen, sind die Probanden 1 und 2, deren Datensätze in den Figuren 5a-c durch die Symbole "Stern" und "Kreis" gekennzeichnet sind.

In einem ersten Schritt fittet die Steuereinheit 2 die Datensätze der Probanden 1 und 2 ("Stern" und "Kreis") in der normierten Darstellung der Fig. 5a jeweils durch eine Fitfunktion (nicht gezeigt). Für jeden Wert der Stimulationspulsamplitude des Nutzers 7 in Fig. 5a bestimmt oder schätzt die Steuereinheit 2 die zugehörige Kraftwahrnehmung des Nutzers 7 dann als ein geeignetes gewichtetes Mittel zwischen den Werten der Kraftwahrnehmungs-Fitkurven an die Datensätze der Probanden 1 und 2 ("Stern" und "Kreis"). Da der MS-Wert und der GU-Wert des Nutzers 7 von den MS- und GU-Werten der Probanden 1 und 2 jeweils den gleichen Abstand haben, wählt die Steuereinheit 2 den Gewichtungsfaktor jeweils als 1, so dass in Fig. 5a die geschätzte Kraftwahrnehmungskurve 11a des Nutzers 7 (gestrichelt) genau zwischen den Kraftwahrnehmungs-Fitkurven der Probanden 1 und 2 ("Stern" und "Kreis") verläuft. Anhand der so bestimmten oder geschätzen Kraftwahrnehmungskurve 11a des Nutzers 7 ermittelt die Steuereinheit 2 im Beispiel der Fig. 5a dann den Wert A₁ der Stimulationspulsamplitude, der beim Nutzer 7 die gewünschte Kraftwahrnehmung von 30 N erzeugt.

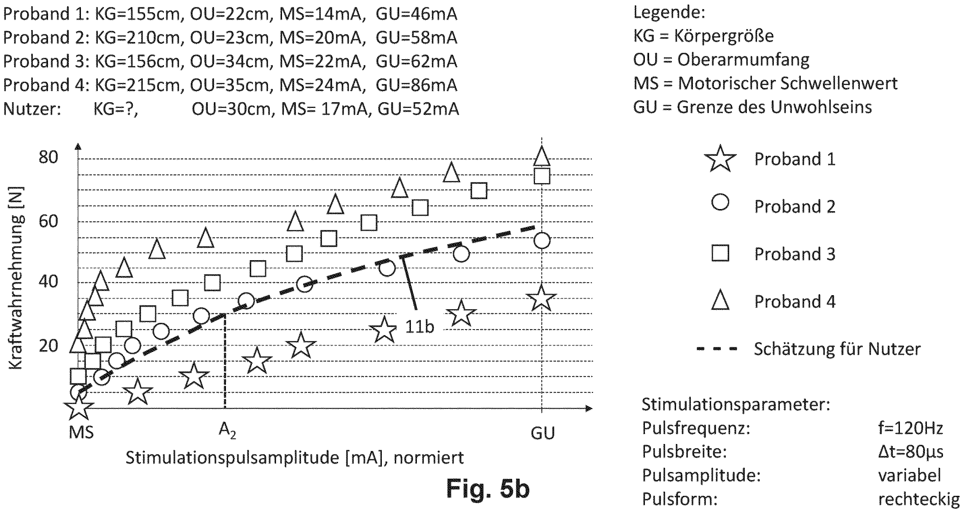

Im in Fig. 5b gezeigten Beispiel bestimmt oder schätzt die Steuereinheit 2 den gesuchten Wert der Stimulationspulsamplitude, der beim Nutzer 7 eine gewünschte Kraftwahrnehmung von 30 N hervorruft, basierend auf den in Fig. 5b enthaltenen Kalibrierungsdaten der Probanden 1 bis 4, basierend auf den Werten von MS und GU des Nutzers 7 und basierend auf dem OU-Wert des Nutzers 7. Da der Steuereinheit 2 für den Nutzer 7 die drei Werte von MS, GU und OU vorliegen, wählt die Steuereinheit 2 zur Bestimmung oder Schätzung des gesuchten Wertes der Stimulationspulsamplitude aus den Kalibrierungsdatensätzen der vier Probanden nur die Kalibrierungsdatensätze derjenigen drei Probanden aus, die dem Kalibrierungsdatensatz des Nutzers 7 im durch die physischen Parameter MS, GU und OU aufgespannten dreidimensionalen Parameterraum am nächsten liegen, wobei die Steuereinheit 2 als Abstandsmaß im MS-GU-OU-Raum die euklidische Metrik verwendet. Die drei Probanden, deren Datensätze im MS-GU-OU-Raum dem Datensatz des Nutzers 7 am nächsten liegen, sind die Probanden 1, 2 und 3, deren Datensätze in den Figuren 5a-c durch die Symbole "Stern", "Kreis" und "Quadrat" gekennzeichnet sind.

In einem ersten Schritt fittet die Steuereinheit 2 die Datensätze der Probanden 1, 2 und 3 ("Stern", "Kreis, "Quadrat") in der normierten Darstellung der Fig. 5b jeweils durch eine Fitfunktion (nicht gezeigt). Für jeden Wert der Stimulationspulsamplitude des Nutzers 7 in Fig. 5b bestimmt oder schätzt die Steuereinheit 2 die zugehörige Kraftwahrnehmung des Nutzers 7 dann als ein geeignetes gewichtetes Mittel zwischen den Werten der Kraftwahrnehmungs-Fitkurven an die Datensätze der Probanden 1, 2 und 3 ("Stern", "Kreis, "Quadrat"). Anhand der so bestimmten oder geschätzen Kraftwahrnehmungskurve 11b des Nutzers 7 ermittelt die Steuereinheit 2 im Beispiel der Fig. 5b dann den Wert A₂ der Stimulationspulsamplitude, der beim Nutzer 7 die gewünschte Kraftwahrnehmung von 30 N erzeugt.

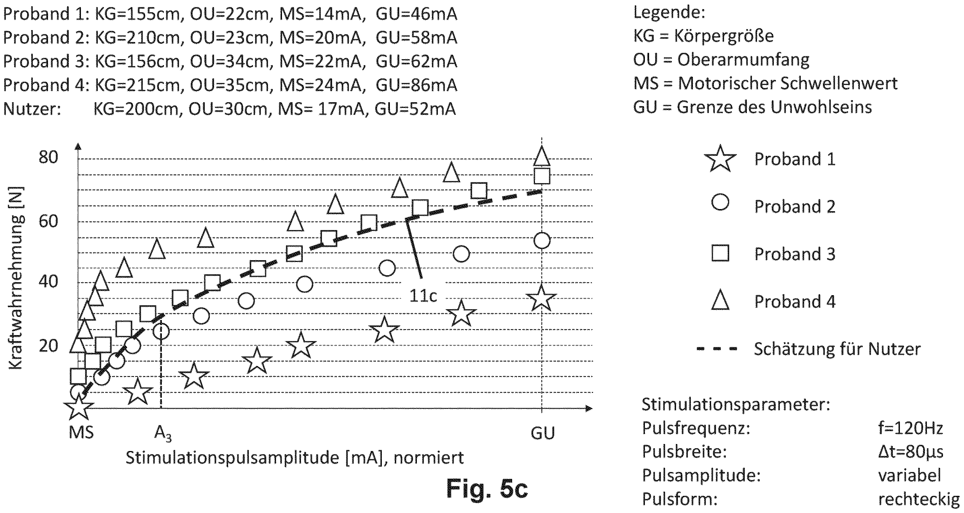

Im in Fig. 5c gezeigten Beispiel bestimmt oder schätzt die Steuereinheit 2 den gesuchten Wert der Stimulationspulsamplitude, der beim Nutzer 7 eine gewünschte Kraftwahrnehmung von 30 N hervorruft, basierend auf den in Fig. 5c enthaltenen Kalibrierungsdaten der Probanden 1 bis 4, basierend auf den Werten von MS und GU des Nutzers 7, basierend auf dem OU-Wert des Nutzers 7 und basierend auf dem KG-Wert des Nutzers 7. Da der Steuereinheit 2 für den Nutzer 7 die vier Werte von MS, GU, OU und KG vorliegen, wählt die Steuereinheit 2 zur Bestimmung oder Schätzung des gesuchten Wertes der Stimulationspulsamplitude die Kalibrierungsdatensätzen aller vier Probanden aus.

In einem ersten Schritt fittet die Steuereinheit 2 die Datensätze der Probanden 1, 2, 3 und 4 ("Stern", "Kreis, "Quadrat", "Dreieck") in der normierten Darstellung der Fig. 5c jeweils durch eine Fitfunktion (nicht gezeigt). Für jeden Wert der Stimulationspulsamplitude des Nutzers 7 in Fig. 5c bestimmt oder schätzt die Steuereinheit 2 die zugehörige Kraftwahrnehmung des Nutzers 7 dann als ein geeignetes gewichtetes Mittel zwischen den Werten der Kraftwahrnehmungs-Fitkurven an die Datensätze der Probanden 1, 2, 3 und 4 ("Stern", "Kreis, "Quadrat", "Dreieck"). Anhand der so bestimmten oder geschätzen Kraftwahrnehmungskurve 11c des Nutzers 7 ermittelt die Steuereinheit 2 im Beispiel der Fig. 5c dann den Wert A₃ der Stimulationspulsamplitude, der beim Nutzer 7 die gewünschte Kraftwahrnehmung von 30 N erzeugt.

## Patentansprüche

1. System (10) zum Stimulieren wenigstens eines Muskels mittels elektrischer Stimulation, umfassend:
wenigstens ein Elektrodenpaar (3) zum Verabreichen elektrischer Stimulation und
eine mit dem wenigstens einen Elektrodenpaar (3) verbindbare Steuereinheit (2) zum Ansteuern des wenigstens einen Elektrodenpaares (3),
wobei die Steuereinheit (2) eingerichtet ist,
ein Elektrodenpaar (3) des wenigstens einen Elektrodenpaares (3) während einer ersten Kalibrierungsphase zum Stimulieren wenigstens eines Muskels wenigstens eines Probanden (1) derart anzusteuern, dass ein erster Stimulationsparameter jeweils wenigstens einen ersten Stimulationsparameterwert annimmt,
ein Elektrodenpaar (3) des wenigstens einen Elektrodenpaares (3) während einer zweiten Kalibrierungsphase zum Stimulieren wenigstens eines Muskels eines von dem Probanden (1) verschiedenen Nutzers (7) derart anzusteuern, dass ein zweiter Stimulationsparameter wenigstens einen zweiten Stimulationsparameterwert annimmt, und
basierend auf dem wenigstens einen ersten Stimulationsparameterwert und basierend auf dem wenigstens einen zweiten Stimulationsparameterwert wenigstens einen dritten Stimulationsparameterwert eines dritten Stimulationsparameters zu bestimmen oder zu schätzen und ein Elektrodenpaar (3) des wenigstens einen Elektrodenpaares (3) während einer Anwendung zum Stimulieren wenigstens eines Muskels des Nutzers (7) derart anzusteuern, dass der dritte Stimulationsparameter den wenigstens einen dritten Stimulationsparameterwert annimmt.

2. System (10) nach einem der vorhergehenden Ansprüche, dadurch gekenzeichnet, dass der erste Stimulationsparameter, der zweite Stimulationsparameter und der dritte Stimulationsparameter jeweils einen der folgenden Parameter umfassen: eine Pulsform elektrischer Stimulationspulse, eine Amplitude elektrischer Stimulationspulse, eine Frequenz elektrischer Stimulationspulse, eine Pulsdauer elektrischer Stimulationspulse, eine Gesamtdauer der Stimulation, eine Gesamtenergie der Stimulation.

3. System (10) nach Anspruch 1 oder 2, dadurch gekenzeichnet, dass der erste Stimulationsparameter gleich dem zweiten Stimulationsparameter und dem dritten Stimulationsparameter ist.

4. System (10) nach Anspruch 3, **dadurch gekennzeichnet, dass** wenigstens einer des wenigstens einen ersten Stimulationsparameterwertes von wenigstens einem des wenigstens einen zweiten Stimulationsparameterwertes verschieden ist.

5. System (10) nach einem der vorhergehenden Ansprüche, **dadurch gekennzeichnet, dass** der wenigstens eine erste Stimulationsparameterwert einen kleinsten Wert des ersten Stimulationsparameters umfasst, der den wenigstens einen Muskel des wenigstens Probanden (1) während der ersten Kalibrierungsphase jeweils zu einer Kontraktion veranlasst, und dass der wenigstens eine zweite Stimulationsparameterwert einen kleinsten Wert des zweiten Stimulationsparameters umfasst, der den wenigstens einen Muskel des Nutzers (7) während der zweiten Kalibrierungsphase zu einer Kontraktion veranlasst; und/oder dass der wenigstens eine erste Stimulationsparameterwert einen größten Wert des ersten Stimulationsparameters umfasst, der den wenigstens einen Muskel des wenigstens einen Probanden (1) während der ersten Kalibrierungsphase jeweils ohne geringfügiges Unwohlsein des Probanden (1) zu einer Kontraktion veranlasst, und dass der wenigstens eine zweite Stimulationsparameterwert einen größten Wert des zweiten Stimulationsparameters umfasst, der den wenigstens einen Muskel des Nutzers (7) während der zweiten Kalibrierungsphase ohne geringfügiges Unwohlsein des Probanden (1) zu einer Kontraktion veranlasst.

6. System (10) nach einem der vorhergenden Ansprüche, **gekennzeichnet durch** eine mit der Steuereinheit (2) verbundene oder verbindbare Eingabeeinheit (4) zum Eingeben wenigstens eines ersten physischen Parameterwertes wenigstens eines physischen Parameters des wenigstens einen Probanden (1) während der ersten Kalibrierungsphase und zum Eingeben wenigstens eines zweiten physischen Parameterwertes wenigstens eines physischen Parameters des Nutzers (7), wobei die Steuereinheit (2) eingerichtet ist, den wenigstens einen dritten Stimulationsparameterwert zusätzlich basierend auf dem wenigstens einen ersten physischen Parameterwert und basierend auf dem wenigstens einen zweiten physischen Parameterwert zu bestimmen oder zu schätzen.

7. System (10) nach Anspruch 6, **dadurch gekennzeichnet, dass** der wenigstens eine physische Parameter des wenigstens einen Probanden (1) und/oder des Nutzers (7) einen, mehrere oder alle der folgenden Parameter umfasst:
- eine Bezeichnung eines stimulierten und/oder zu stimulierenden Muskels,
- Körpergröße,
- Körpergewicht,
- Lebensalter,
- Geschlecht,
- Körperfettanteil,
- Knochenmasse,
- Körperflüssigkeitsanteil,
- Muskelmasse,
- wenigstens ein Größenmaß wenigstens eines Körperteils,
- eine maximal generierbare Kraft durch willentliche (oder willkürliche) Aktivierung eines Muskels,
- wenigstens einen Wert einer infolge der Stimulation mit dem wenigstens einen ersten Stimulationsparameterwert während der ersten Kalibrierungsphase durch den wenigstens einen Muskel des Probanden (1) erzeugten Kraft,
- wenigstens einen Wert einer infolge der Stimulation mit dem wenigstens ersten Stimulationsparameterwert während der ersten Kalibrierungsphase von dem Probanden (1) empfundenen Kraft,
- wenigstens einen Wert einer infolge der Stimulation mit dem wenigstens einen zweiten Stimulationsparameterwert während der zweiten Kalibrierungsphase durch den wenigstens einen Muskel des Nutzers (7) erzeugten Kraft,
- wenigstens einen Wert einer infolge der Stimulation mit dem wenigstens einen zweiten Stimulationsparameterwert während der zweiten Kalibrierungsphase von dem Nutzer (7)empfundenen Kraft.

8. System (10) nach einem der vorhergenden Ansprüche, **dadurch gekennzeichnet, dass** die Steuereinheit (2) eingerichtet ist, den wenigstens einen dritten Stimulationsparameterwert mittels maschinellen Lernens zu bestimmen.

9. System (10) nach einem der vorhergehenden Ansprüche, **dadurch gekennzeichnet, dass** die Steuereinheit (2) eingerichtet ist, basierend auf dem wenigstens einen ersten Stimulationsparameterwert und basierend auf dem wenigstens einen zweiten Stimulationsparameterwert eine Tabelle und/oder eine mathematische Vorschrift zu erzeugen, die jedem einer Vielzahl von Stimulationsparameterwerten des dritten Stimulationsparameters jeweils einen Wert einer Kraft oder einer Muskelkontraktion zuordnet, die beim Stimulieren des wenigstens eines Muskels des Nutzers (7) während der Anwendung mit dem jeweiligen Stimulationsparameterwert des dritten Stimulationsparameters durch diesen wenigstens einen Muskel des Nutzers (7) generierbar ist und/oder durch den Nutzer (7)empfindbar ist, wobei die Steuereinheit (2) eingerichtet ist, den wenigstens einen dritten Stimulationsparameterwert basierend auf Einträgen der Tabelle oder basierend auf der mathematischen Vorschrift zu bestimmen oder zu schätzen.

10. System (10) nach Anspruch 6 oder 7 und nach Anspruch 9, **dadurch gekennzeichnet, dass** die Steuereinheit (2) eingerichtet ist, die Tabelle und/oder die mathematische Vorschrift ferner basierend auf dem wenigstens einen ersten physischen Parameterwert des wenigstens einen Probanden (1) und basierend auf dem wenigstens einen zweiten physischen Parameterwert des Nutzers (7) zu erzeugen oder zu schätzen.

11. System (10) nach einem der vorhergehenden Ansprüche, **dadurch gekennzeichnet, dass** die Steuereinheit (2) eingerichtet ist, den wenigstens einen dritten Stimulationsparameterwert mittels eines k-nächste-Nachbarn-Algorithmus zu bestimmen oder zu schätzen.

12. System (10) nach einem der vorhergehenden Ansprüche, **gekennzeichnet durch** ein mit der Steuereinheit (2) verbundenes oder verbindbares Bewegungs-Erfassungssystem (5) zum Erfassen einer Haltung und/oder einer Bewegung wenigstens eines Teils eines Körpers eines Probanden (1) und/oder eines Nutzers (7), wobei die Steuereinheit (2) eingerichtet ist, den wenigstens einen dritten Stimulationsparameterwert zusätzlich basierend auf einer durch das Bewegungs-Erfassungssystem (5) erfassten Haltung und/oder Bewegung wenigstens eines Teils des Körpers des Nutzers (7) zu bestimmen oder zu schätzen.

13. System (10) nach einem der vorhergehenden Ansprüche, **gekennzeichnet durch** wenigstens eine mit der Steuereinheit (2) verbundene oder verbindbare Schnittstelle (6) zum Empfangen wenigstens eines Fahrzeugparameterwertes wenigstens eines Fahrzeugparameters eines Fahrzeugs, z. B. eines Kraftfahrzeugs oder eines Flugzeugs, in dem das System (10) und der Nutzer (7) anordenbar sind, wobei die Steuereinheit (2) eingerichtet ist, den wenigstens einen dritten Stimulationsparameterwert zusätzlich basierend auf dem wenigstens einen Fahrzeugparameterwert zu bestimmen.

14. System (10) nach Anspruch 13, **dadurch gekennzeichnet, dass** der wenigstens eine Fahrzeugparameter einen, mehrere oder alle der folgenden Parameter umfasst:
- eine Fahrzeuggeschwindigkeit,
- eine Fahrzeugbeschleunigung,
- eine Fahrzeugmasse,
- eine Fahrzeuglage,
- eine Fahrzeugreisehöhe,
- einen Strömungswiderstand des Fahrzeugs,
- einen Umgebungsluftdruck,
- eine Umgebungstemperatur,
- einen Steuerparameter zum Beeinflussen der Fahrzeuggeschwindigkeit, der Fahrzeugbeschleunigung, der Fahrzeuglage und/oder der Fahrzeugreisehöhe.

15. Verfahren zum Ansteuern eines Elektrodenpaares (3), insbesondere zum Stimulieren wenigstens eines Muskels mittels elektrischer Stimulation, umfassend die Schritte:
während einer ersten Kalibrierungsphase Ansteuern wenigstens eines Elektrodenpaares (3) zum Stimulieren wenigstens eines Muskels eines oder mehrerer Probanden (1) derart, dass ein erster Stimulationsparameter wenigstens einen ersten Stimulationsparameterwert annimmt,
während einer zweiten Kalibrierungsphase Ansteuern wenigstens eines Elektrodenpaares (3) zum Stimulieren wenigstens eines Muskels eines von dem oder den Probanden (1) verschiedenen Nutzers (7) derart, dass ein zweiter Stimulationsparameter wenigstens einen zweiten Stimulationsparameterwert annimmt,
Bestimmen oder Schätzen wenigstens eines dritten Stimulationsparameterwertes eines dritten Stimulationsparameters basierend auf dem wenigstens einen ersten Stimulationsparameterwert und basierend auf dem wenigstens einen zweiten Stimulatioparameterwert und
während einer Anwendung Ansteuern wenigstens eines Elektrodenpaares (3) zum Stimulieren wenigstens eines Muskels des Nutzers (7) derart, dass der dritte Stimulationsparameter den wenigstens einen dritten Stimulationsparameterwert annimmt.
